(19) Europäisches Patentamt
European Patent Office
Office européen des brevets

(11) **EP 4 760 729 A1**

(12) **EUROPEAN PATENT APPLICATION**

(43) Date of publication:
**17.06.2026 Bulletin 2026/25**

(21) Application number: **24218774.8**

(22) Date of filing: **10.12.2024**

(51) International Patent Classification (IPC):
**G16H 10/40** (2018.01)   **B01L 3/00** (2006.01)
**G01N 35/00** (2006.01)   **G16H 40/63** (2018.01)

(52) Cooperative Patent Classification (CPC):
**G16H 10/40; B01L 3/545; G01N 35/00732;**
**G16H 40/63;** B01L 2300/022; B01L 2300/024;
G01N 2035/00673; G01N 2035/00811;
G01N 2035/00851

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Roche Diagnostics International AG**
**6343 Rotkreuz (CH)**

(72) Inventors:
• **BUERGISSER, Reto Alois**
  **6343 Rotkreuz (CH)**
• **HERNANDEZ ARIETA, Alejandro**
  **6343 Rotkreuz (CH)**

(74) Representative: **Mewburn Ellis LLP**
  **Aurora Building**
  **Counterslip**
  **Bristol BS1 6BX (GB)**

(54) **AUTOMATED LABORATORY INSTRUMENT**

(57) An automated laboratory instrument for analysing biological samples. The automated laboratory instrument comprising: a plurality of reagent container holders, each configured to hold a reagent container containing reagent for use in the processing of a biological sample and including a tag formed as a part thereof; a plurality of reagent container tag readers, each associated with a respective reagent container holder, and configured to read contents of the tag of a reagent container held within the respective reagent container holder; and a controller (101), configured to: cause one of the reagent container tag readers to read a timestamp from the tag of the reagent container; obtain a system timestamp; and update the timestamp stored in the tag utilising the obtained system timestamp.

Fig. 1

**Description**

*Field of disclosure*

The present disclosure relates to an automated laboratory instrument, a computer-implemented method, and a computer-readable storage medium.

*Background*

In analytical laboratories, and in particular in-vitro diagnostic laboratories, a multitude of analyses on biological samples can be executed in order to determine physiological or biochemical states of patients.

Most diagnostic tests can be carried out with ready-to-use reagents that have an associated shelf-life. For example, the cobas OMNI which contains lysis buffer, diluent, wash reagent, and magnetic glass particles and other reagent cassettes which provides test specific reagents. Many of the reagents stored in a reagent container are in liquid form, and this can limited the shelf-life of the consumable.

If a reagent container has expired, or has been improperly stored, it is important that the instrument be able to recognise this and act accordingly.

The present disclosure has been devised in light of the above considerations.

*Summary of the Invention*

Broadly, the present disclosure provides an automated laboratory instrument which is configured to track the time that reagent containers have been within the machine. Further, in some examples, this tracked time is calibrated according to the conditions in which the reagent container has been held.

Accordingly, there is provided, in embodiments of a first aspect, an automated laboratory instrument for analysing biological samples. The automated laboratory instrument comprises a plurality of reagent container holders, each configured to hold one or more reagent containers for use in the processing of a biological sample and including a tag formed as a part thereof. The automated laboratory instrument also comprises a plurality of reagent container tag readers, each associated with a respective reagent container holder, and configured to read contents of the tag of a reagent container held within the respective reagent container holder. The automated laboratory instrument also comprises a controller, configured to: cause one of the reagent container tag readers to read a timestamp from the tag of the reagent container; obtain a system timestamp; and update the timestamp stored in the tag utilising

Advantageously, this allows the automated laboratory instrument to track the time a given regent container has spent within the instrument.

The automated laboratory instrument (e.g., analytical system) may be an automated molecular work area or device for performing IVD (in vitro diagnostic) tests on biological samples such as Polymerase Chain Reaction (PCR) based Nucleic Acid Testing (NAT). The automated laboratory instrument may comprise an onboard storage and refrigeration system to enable ready access to and maintain an inventory of consumables and reagents for performing the tests.

The reagent container may be a single container containing a single reagent, or may be a reagent cassette containing a number of reagents or consumables (e.g., as a kit or set for performing a particular process in the instrument).

The controller may be a processor or system manager configured to receive test orders, determine workflows for fulfilling each of the test orders, and configured the automated laboratory instrument to perform the workflows. For example, the controller may receive status information about the testing hardware, consumables, and reagents of automated laboratory instrument so as to determine an efficient workflow from sample processing to result interpretation.

The plurality of reagent container holders may be bays or other parts or modules of the automated laboratory instrument configured to hold one or more reagent containers. For example, a reagent container holder may be an input draw or unit into which reagent containers can be loaded for ingress into the instrument. The reagent container holders may include one or more interim reagent storage hardware units, one or more long-term reagent storage hardware units, and one or more testing units (at which a process is to be performed using the consumable stored within the reagent container).

One or more, or all, of the plurality of reagent container tag readers may be radio-frequency identification tag readers (RFID). Alternatively, one or more, or all, of them may be near-field communication (NFC) readers and/or Bluetooth low energy (BLE) readers.

The controller may be further configured to read an elapsed time value from the tag of the reagent container.

The timestamp may indicate the time to the level of minutes, seconds, or milliseconds. The elapsed time value may indicate the time to the level of minutes, seconds, or milliseconds. Preferably the timestamp and elapsed time values are indicated to the level of minutes (and rounded up, where needed).

The controller may be further configured to update the elapsed time value obtained from the tag of the reagent container. The controller may be configured to obtain a temperature of the reagent container or reagent container holder in which the reagent container is located, and the update to the elapsed time value stored in the tag may be based on the obtained temperature. For example, one or more, or all, of the reagent container holders may include a temperature sensor, the controller being configured to obtain a reading from the temperature sensor when also causing the respective tag reader to read the tag. The controller may be configured to apply a temperature factor to a difference between the obtained system timestamp and the timestamp stored in the tag prior to updating the elapsed time value when the obtained temperature is within a first predetermined range of temperatures. For example, where the obtained temperature is greater than a first value but less than a second value (larger than the first), a factor greater than 0 but less than 1 may be applied to the difference. The controller may be configured to update the elapsed time value from the tag of the reagent container only when a temperature of the reagent container is greater than a lower threshold. For example, the temperature factor may be set to zero when the temperature is lower than threshold (such that the value of the difference is also set to zero). Alternatively, the controller may be configured to perform no update action when the temperature is lower than the lower threshold. If the obtained temperature is within a second predetermined range of temperatures, the controller may be configured to update the elapsed time value based only on the difference between the timestamp read from the tag prior to the update and the obtained system timestamp (that is, a temperature factor is not used or is set to one so as to not impact the calculation).

As an example, the controller may be configured to not update the elapsed time value when the temperature of the reagent container is less than or equal to 8°C. If the temperature is below 2°C, a frozen or invalid flag may be set by the controller on the tag of the container (as typically reagent containers cannot be used if exposed to temperatures below 2°C). If the temperature is greater than 8°C, but less than or equal to 25°C, the controller may be configured to use a temperature factor of 0.66 (or 66%). This would reduce the time to be added to the elapsed time value by 1/3. If the temperature is greater than 25°C, the controller may be configured to disregard the temperature factor (or set the temperature factor to 1). This would mean that the time added to the elapsed time value is the difference between the system timestamp and the previously read timestamp from the tag of the container.

The controller may be configured, when a reagent container is placed or located in an instrument ingress reagent container holder of the plurality of reagent container holders, to set a usage flag stored in the tag of the reagent container. The ingress reagent container holder may be a first of the plurality of reagent container holders in a processing line, for example from ingress to the machine to (ultimately) the waste collection or output drawer. The usage flag set may be selected from a plurality of usage flags, based on information held by the controller relating to the automated laboratory instrument. For example, the usage flag may be selected or set to denote that the reagent container has been placed in a particular make or model of automated laboratory instrument. This can avoid the reagent container being transferred to another automated laboratory instrument, where it's use in a previous one precludes its use in a future instrument (for example due to incompatibilities in the storage and/or handling of the reagent container). The controller may be further configured, when a reagent container is placed or located in the ingress reagent container holder, to read any set usage flags and to further determine whether the usage flag that has been set is compatible with the automated laboratory instrument.

The controller may be further configured, when a reagent container is stored in a long-term reagent storage, to reset or remove the timestamp stored in the tag. When the reagent container is stored in the reagent storage unit, the temperature is controlled to be under 8°C. In this situation, the reagent stability does not change and therefore, to avoid issues if the reagent was removed by hand, the tracking timestamp can be reset.

The controller may be further configured to determine whether the reagent container is invalid based on an elapsed time value read from the tag of the reagent container, and where it is, the controller may be configured to set an invalid flag stored on the tag of the reagent container. The controller may, for example, look-up or read from the tag shelf-life value for the reagent container, and where the elapsed time has exceeded the shelf-life value, determine the reagent container to be invalid. The controller may be configured to reject reagent containers having a set invalid flag.

The controller may be configured to mirror at least some, or all, of the contents of the tag of the reagent container in memory and/or storage of the automated laboratory instrument. This can allow updates to the tags of the reagent containers to be tracked without requiring them to be moved to the reagent container tag reader for every state change.

The controller may be further configured to monitor a temperature of one or more of the reagent container holders, and may be configured to set an invalid or overtemperature flag stored on the tag of a reagent container stored in that reagent container holder when the temperature of the reagent container holder exceeds an acceptable upper temperature limit or is lower than an acceptable lower temperature limit.

The controller may be further configured to monitor the removal of a regent container from the automated laboratory instrument, and upon return of the same reagent container to the automated laboratory instrument, to update an elapsed time value stored on the tag of the reagent container to reflect the time during which the reagent container was removed from the automated laboratory instrument. For example, by using the latest timestamp on the reagent container (it having been updated immediately prior to being removed from the instrument), or from reading a unique reagent container ID (stored on the tag) at the removal and insertion points, and using system timestamps associated with those two events.

The controller may be further configured to determine from an elapsed time value read from the tag of a reagent container, whether the reagent container can be used in a planned run. For example, the controller may receive or have access to a schedule of runs (e.g., one or more test orders to be performed by the instrument), and can determine whether the reagent container will have expired by the time the corresponding run in which it is to be used is to be performed. If so, the controller may notify an operator, or automatically substitute the to-expire reagent container with one which will be usable at the required time.

The controller may be configured, during an initialisation phase, to detect the presence of one or more reagent containers within an interim reagent storage module, and for each of the detected reagent containers:

> obtain the system stamp;
> read the timestamp and an elapsed time value from the tag of the reagent container;
> update the elapsed time value stored on the tag; and
> update the timestamp stored on the tag.

In a second aspect, embodiments of the disclosure provide a computer-implemented method comprising the steps, on an automated laboratory instrument, of:

> reading, via a reagent container tag reader, a timestamp from a tag of a reagent container held in one of a plurality of reagent container holders;
> obtain a system timestamp; and
> update the timestamp stored in the tag utilising the obtained system timestamp.

The method may be performed by the controller of the automated laboratory instrument. The controller may perform any part of any of the optional features set out with reference to the first aspect.

A computer-readable medium comprising instructions which when executed by a computer, cause the computer to perform the computer-implemented method of the second aspect.

The invention includes the combination of the aspects and preferred features described except where such a combination is clearly impermissible or expressly avoided.

### *Summary of the Figures*

Embodiments and experiments illustrating the principles of the invention will now be discussed with reference to the accompanying figures in which:

> **Fig. 1** shows an automated laboratory instrument;
> **Fig. 2** shows a flow chart;
> **Fig. 3** shows a flow chart;
> **Fig. 4** shows a flow chart;
> **Fig. 5** shows a flow chart;
> **Fig. 6** shows a flow chart;
> **Fig. 7** shows a flow chart;
> **Fig. 8** shows a flow chart; and
> **Fig. 9** shows a flow chart.

### *Detailed Description of the Invention*

Aspects and embodiments of the present invention will now be discussed with reference to the accompanying figures. Further aspects and embodiments will be apparent to those skilled in the art. All documents mentioned in this text are incorporated herein by reference.

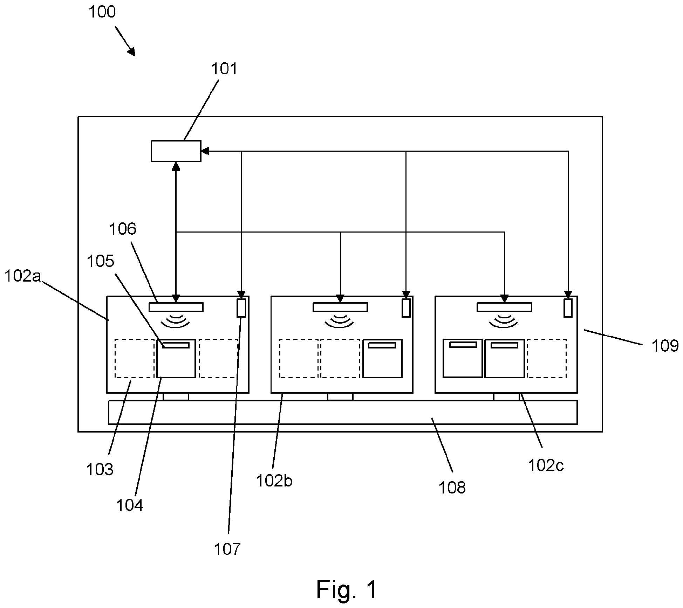

**Fig. 1** shows an automated laboratory instrument 100. The automated laboratory instrument includes a plurality of reagent container holders 102a - 102c, whilst three are shown of course the number may be higher or lower. Each of the reagent container holders has one or more slots 103, indicated by the dashed lines, into which reagent containers 104 can be placed. Each reagent container 104 includes a tag 105 as discussed previously, in this example an RFID tag which can be read by a reader 106. Each of the reagent container holders has such a reader 106, which can communicate with the reagent containers located within it. In some examples, it may be that one of the slots 103 of the reagent container is the one in which the tag of a reagent container can be read, and so the reagent container may be moved to that slot in order for the tag to be read. In other examples, the reader 106 may be able to read the tags from all of the reagent containers held within its reagent container holder.

Each reader 106 is connected to a controller 101, which can perform numerous actions as discussed in detail below. At least some, and in this example all, of the reagent container holders 102a-102c also have a temperature sensor 107, which is connected to the controller (either directly, or through one or more intermediary components such as the reader 106) so as to report the temperature within the respective reagent holder. Each reagent container holder is also connected to a conveying system 108, which is configured to move regent containers between the reagent container holders. The controller 101 includes, or has access to, a system clock which provides the time.

In this example, the regent container holder 102a is an ingress reagent container holder, and it is into this that new reagent containers 104 are loaded (and from which they may be removed). Reagent container holder 102b is an interim reagent container holder, such that the reagent containers held therein may be used for analytical runs to process samples, and reagent container holder 102c is a long-term storage reagent container holder. Here, the long-term storage reagent container holder is refrigerated and so cooled (relative to the other holders).

Generally, the controller is configured to use the readers 106 to read the tag 105 of a reagent container 104 when that reagent container is moved into a given reagent container holder 102. The controller is configured to read a timestamp from the tag, obtain the current system time, and use this to update the timestamp stored on the tag. The tag also includes an elapsed time value, indicating how much time the reagent container has spent in the instrument. However, this time is adjusted based on the condition in which the reagent container has been stored. This elapsed time value (also referred to as an on-board time tracking value), is calculated in minutes (rounded up) by measuring the difference between the system timestamp and the timestamp stored on the reagent container's tag (which may be referred to as an RFID label). This can be performed at a transaction time in the instrument (i.e., when the instrument is interacting with the reagent container).

Depending on the temperature range, different update rules can be applied:

    a. Temperature between 2-8°C: no tracking;

    b. Temperature between >8°C - 25°C, the reagent container is determined to be unrefrigerated, and the elapsed time value is calculated by multiplying the measured time by a cooled on-board time factor (e.g., 66% or 0.66);

    c. Temperature between >25°C - 37°C: measured time (no time factor applied; and

    d. Temperature >37°C or <2°C: tracking is stopped, and the reagent container is marked with an invalid temperature flag on detection

The on-board time is calculated by measuring the difference between the instrument software timestamp (the system timestamp, as obtained by the controller) and the timestamp stored in the on-boardTimeStamp field in the RFID label of the reagent container. The result in minutes (rounded up) is stored in the on-boardTimeTracking field in the RFID label.

The controller in the instrument 100 is configured to start tracking the on-board time when the reagent container is exposed to a temperature over 8°C. This will happen, for example, when the container is removed from the reagent storage for use or if the reagent storage temperature is out of the expected temperature band.

When the reagent container is removed from the reagent storage for processing, the on-boardTimeStamp is updated with the current software timestamp and the Configuration1_Used flag or field is set to true.

For every transition step on the instrument, when the reagent container reaches a scanner station (e.g., an RFID station) in a hardware unit, the update is performed as follows if an update is required:

    a. Get current system timestamp;

    b. Calculate difference (in minutes, rounded up) with stored timestamp;

    c. Apply cooled on-board time factor if the temperature is > 8°C and < 25°C (e.g., in interim reagent storage);

    d. Update on-boardTimeTracking; and

    e. Update on-boardTimeStamp with the current time stamp.

Only when the reagent container is stored back in long-term storage, the update procedure is performed as follows:

    a. Get current system timestamp;

b. Calculate difference (in minutes, rounded up) with stored timestamp;

c. Update on-boardTimeTracking; and

d. Update on-boardTimeStamp with zero (i.e., remove the on-board TimeStamp).

Due to the relatively small number of RFID stations in the reagent storage and interim reagent storage hardware units (the reagent container holders), the software units (e.g., the controller) can cache the data in memory (RAM) and persist the data in the file system, so that the updates can be tracked without requiring the reagent containers to be moved to an RFID reader for every state change.

A number of use cases will now be discussed, the first considers loading a reagent container into the instrument.

*New Container*

A new container will have on-boardTimeTracking and on-boardTimeStamp set to zero. The tag will not be updated, and the Configuration1_Used flag is not set. Since the container is stored in reagent storage with a temperature lower than 8°C, the container on-board time does not increase (and correspondingly, the onboard stability timer discussed below does not decrease).

*Used Container*

A used container will have on-boardTimeTracking > zero, an on-boardTimeStamp set to zero, and a Configuration1_Used flag already set. Since the container is stored in the reagent storage with a temperature lower than 8°C, the container on-board time does not increase.

*Container removed manually from the instrument outside of reagent storage*

In the case that a container has been manually removed from the instrument (i.e., a cassette deck or the interim reagent storage) due to a power loss or manual clean-up of the instrument, the reagent container, when it is manually reloaded, will have on-boardTimeTracking > zero, on-boardTimeStamp different (!=) zero, and Configuration1_Used flag set.

The controller will update the on-board time as follows:

a. Get current system timestamp;

b. Calculate difference (in minutes, rounded up) with the timestamp stored in the tag;

c. Update on-boardTimeTracking (existing value with added delta); and

d. Set on-boardTimesTamp to zero.

This scenario assumes the container has not been exposed to temperatures outside the acceptable range of either >37°C or <2°C.

*Automatic removal from storage*

When a container is removed from storage for processing, the on-board time tracking is started by updating the on-boardTimeStamp with the current timestamp, and the Configuration1_Used (instrument model specific) flag setting will be set.

*Automatically loading a reagent container in the interim reagent storage*

When a container is loaded into the interim reagent storage, the on-board time tracking is updated according to the update process as follows:

a. Get current system timestamp;

b. Calculate difference (in minutes, rounded up) with timestamp stored on tag;

c. Apply cooled on-board time factor if the temperature was consistently ≤ 25°C (interim reagent storage);

d. Update on-boardTimeTracking; and

e. Update on-boardTimeStamp with current timestamp.

*Automatically unloading a reagent container from the interim reagent storage*

When a container is unloaded from the interim reagent storage, the on-board time tracking is updated according to the update process as follows:

a. Get current system timestamp;

b. Calculate difference (in minutes, rounded-up) with timestamp stored on tag;

c. Apply cooled on-board time factor (66%) if the temperature was consistently ≤ 25°C;

d. Update on-boardTimeTracking; and

e. Update on-boardTimeStamp with the current timestamp.

*Automatically storing back a container to the reagent storage*

When a container is stored back into the reagent storage from use in a transfer or processing module, the on-board time tracking is updated as follows:

a. Get current system timestamp;

b. Calculate difference (in minutes, rounded up) with timestamp stored ont ag;

c. Update on-boardTimeTracking; and

d. Update on-boardTimeStamp to zero.

*Control container in consumable park*

The on-board time is not updated in this position, and instead it will be updated when the container is stored back in the reagent storage as described above.

*Container data update in UI*

The instrument controller (for example, via one or more software units), working with the reagent container scanners, can provide the information necessary for the user interface of the instrument to calculate the current on-board time of the containers in the instrument. A UI API can calculate the status of a specific container using the following formula:

Remaining on-board stability = (on-boardStability - (on-boardTimeTracking + ((Current system timestamp - timestamp read from tag) * cooled factor)))

If the container timestamp is zero (the container is in storage), the cooled factor can be set to zero, so only the accumulated on-board time is used. The on-boardStability is the shelf life of the container, that is, how long the container has before it expires and is unusable.

*Container data update for run planning*

The method discussed above can be adapted to assess if a container will be available for future run.

Remaining on-board stability = (on-boardStability - (on-boardTimeTracking + ((Future timestamp - timestamp read from tag) * cooled factor)))

If the container timestamp is zero (the container is in storage), the cooled factor can be set to zero, so only the accumulated on-board time is used. The future timestamp can be selected to correspond with the time in the future that the reagent container is to be used in the future run.

Exception scenarios

It is useful for the instrument to persist in memory (or long-term storage) the contents of the containers tags, to track them at all times. This enables the following exception scenarios.

*Reagent storage temperature out of regular range*

If the reagent storage temperature is out of its regular range (refrigerated (2°C - 8°C)), the instrument will update the persisted data as follows:

 a. Get current system timestamp;

 b. Update the on-boardtimeStamp in the cached / persisted data with the current timestamp;

 c. The cooled on-board time factor is updated according to the temperature range; and

 d. The reagent storage reports the change in storage positions with the updated data.

The tags of the stored containers are updated with the cached data when the container is unloaded (e.g., when a manual unload is initiated by the user via the UI) or when the container is removed from the storage to be used in the transfer or processing module (e.g., unloaded for transfer), as follows:

When the container is moved to a reagent container tag reader:

If temperature >8°C and $\leq$ 25°C:

 a. Get current system timestamp;

 b. Calculate difference (in minutes, rounded up) with the persisted data timestamp for the container;

 c. Apply cooled on-board time factor (e.g., 66%);

 d. Update on-boardTimeTracking; and

 e. Update on-boardTimeStamp with the current system timestamp.

If temperature >25°C and <37°C:

 a. Get current system timestamp;

 b. Calculate difference (in minutes, rounded up) with the persisted data timestamp;

 c. Apply cooled on-board time factor (100%); and

 d. Update on-board time tracking.

If the operator unloads the container using the instruments UI:

 a. Clear the on-boardTimeStamp to allow external storage of the container

If the instrument transfers the container for processing:

 a. Update the on-boardTimeStamp with the current timestamp.

*Reagent storage temperature out of operational range - too high temperature (>37°C)*

The controller, e.g., via instrument software, reports (for example to the instrument UI) that the reagent storage temperature is out of the operational range and updates the persisted data with the overtemperature and invalid container flags. The instrument UI can then request that an operator unload the containers, and during the unload process the container will be moved to a reagent container tag reader associated with the reagent storage so that the container tags can be updated and moved to the drawer for the operator to remove.

*Reagent storage temperature out of operational range - too low temperature (<2°C)*

The controller, e.g., via instrument software, reports (for example to the instrument UI) that the reagent storage is out of the operational range and update the persisted data with the invalid container flag. The tags on the containers are updated as part of the manual unload process to remove the containers from the system.

*Shutting down instrument - invalidated containers*

The controller will not allow the instrument to be shut down where containers are loaded in the reagent storage or the interim reagent storage.

*Interim reagent storage temperature out of cooled storage range*

If the interim reagent storage temperature is out of the cooled storage range (e.g., >25°C and ≤ 37°C, the cooled on-board factor is set to 1. When the container is removed from the interim reagent storage, the tag on the container will be updated with the time difference between the last update timestamp and the current timestamp with a cooled on-board factor of 1.

The controller will communicate to the rest of the instrument software the updated data with the cooled on-board factor set to 1, and the contents of the updated tag.

*Interim reagent storage temperature out of operational range (>37°C)*

The controller, e.g., via instrument software, reports (for example to the instrument UI) that the temperature of the interim reagent storage is out of the operational range, and also update the persisted data with the overtemperature and invalid container flags.

The controller, e.g., via instrument software, then updates the content of the container's tag by updating the persisted data, moving the container to a reagent container tag reader, and updating the tag with the overtemperature flag and invalid container flags. The container is then moved to solid waste or to the drawer for removal.

*Containers found in the interim reagent storage during initialisation*

If during the initialisation of interim reagent storage, the controller detects that there are containers inside the interim reagent storage, the controller will update the on-board time during the inventory as follows:

    a. Get current system timestamp;

    b. Calculate difference (in minutes, rounded up) with the stored timestamp in the tag of the container;

    c. Update the on-boardTimeTracking; and

    d. Update the on-boardTimeStamp with the current timestamp.

The controller can then remove the containers from the interim reagent storage and load them into the reagent storage.

*Containers found during initialisation in the reagent storage after a restart*

In the case that the instrument restarts, containers are not exposed to higher temperatures as the front door of the reagent storage remains closed. The controller can perform a check of the known positions, and report back the content of the persisted data.

*Containers found during initialisation in the reagent storage after power loss*

If containers are present in the reagent storage, the controller performs a full inventory and updates the on-board time according to the temperature log (if available) whilst the system was without power, and set the Configuration1_Used flag to true.

there is no available temperature log, the containers on-board time will be updated according to If the current temperature of the reagent storage during start-up and the current timestamp of the instrument.

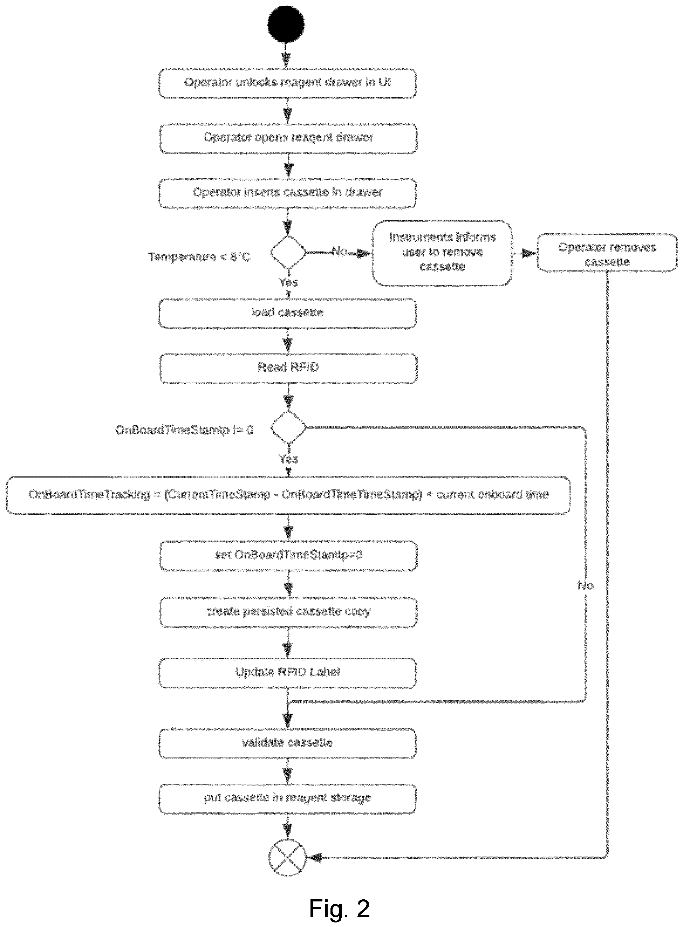

**Fig. 2** shows a flow chart of a method performed when manually loading containers (referred to here as cassettes) into the instrument. First, the operator unlocks the reagent drawer using the instruments user interface (e.g., touchscreen). This allows the operator to open the reagent drawer, and insert a cassette into the drawer. The controller then determines if the temperature of the drawer is less than 8°C. If not, the instrument informs the user via the UI to remove the cassette. The operator can then do so, and the process ends.

If the temperature is less than 8°C, the instrument loads the cassette and reads the RFID label / tag. The controller then determines if the onBoardtimeStamp is not equal to zero (!=0). If it is not, (so onBoardTimeStamp=0), the method moves to the controller validating the cassette and placing it in reagent storage.

If it is (and so onBoardTimeStamp has a value different to zero), the method moves to the controller calculating the onboardTimeTracking as being equal to (CurrentTimeStamp - OnBoardTimeStamp) + current onBoardtime. Next, the onBoardTimeStamp on the label is set to zero, and the persisted cassette copy is created on the instrument. The RFID label is then updated, and the method moves to validate the cassette and place it in the reagent storage.

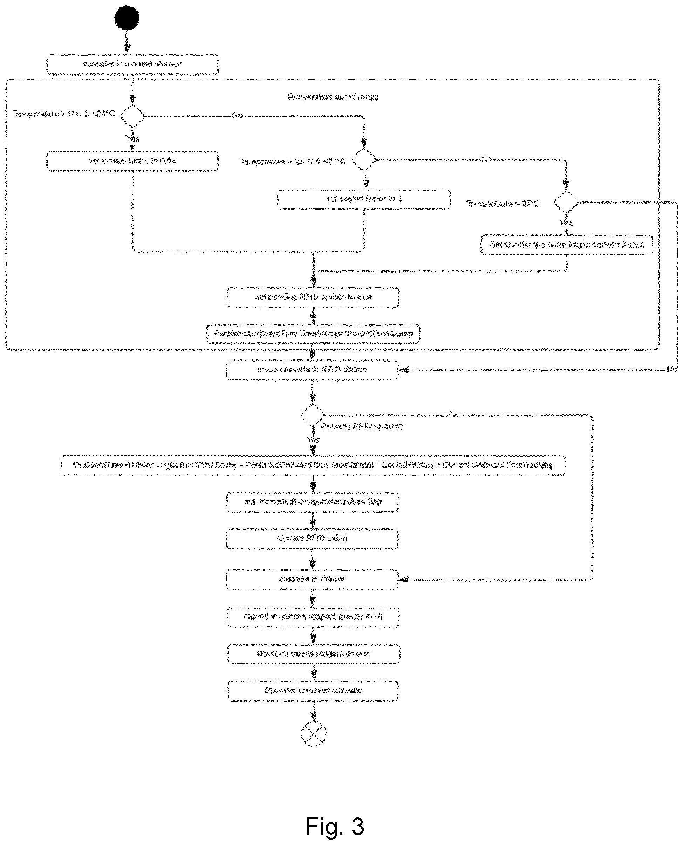

**Fig. 3** shows a flow chart of a method performed when the operator unloads a cassette using the instrument UI. The method begins with a cassette being in the reagent storage. A first determination is made by the controller, as to whether the temperature of the reagent storage is >8°C and <24°C. If so, the cooled factor is set to 0.66. If not, the controller determines if the temperature is >25°C and <37°C, if so, the cooled factor is set to 1. If not, the controller then determines if the temperature is >37°C, and if so the overtemperature flag is set in the persisted data for that cassette. If not, the method moves to a step of moving the cassette to an RFID station.

However, if the cooled factor is set (to 0.66 or 1) or the overtemperature flag is set, the method moves to a step in which a pending RFID update flag is set to true. Then, the PersistedOnBoardTimeStamp is set to the CurrentTimeStamp (the system timestamp). The method then moves to the step discussed above, where the cassette is moved to an RFID station. After it has, a determination is made as to whether the pending RFID update flag has been set. If not, the method moves to a step in which the cassette is locate in a drawer.

If the pending RFID update flag has been set (Yes), the method moves to calculating the onBoardTimeTracking = ((CurrentTimeStamp - PersistedOnBoardTimeStamp) * CooledFactor) + Current onBoardTimeTracking. The method then moves to a step where the PersistedConfiguration1Used flag is set, and then the RFID label is updated with these values and flags.

The method then moves to the same step discussed above, where the cassette is located in the drawer. The operator can then unlock the reagent drawer using the instrument UI, and then open it. The method ends with the operator removing the cassette.

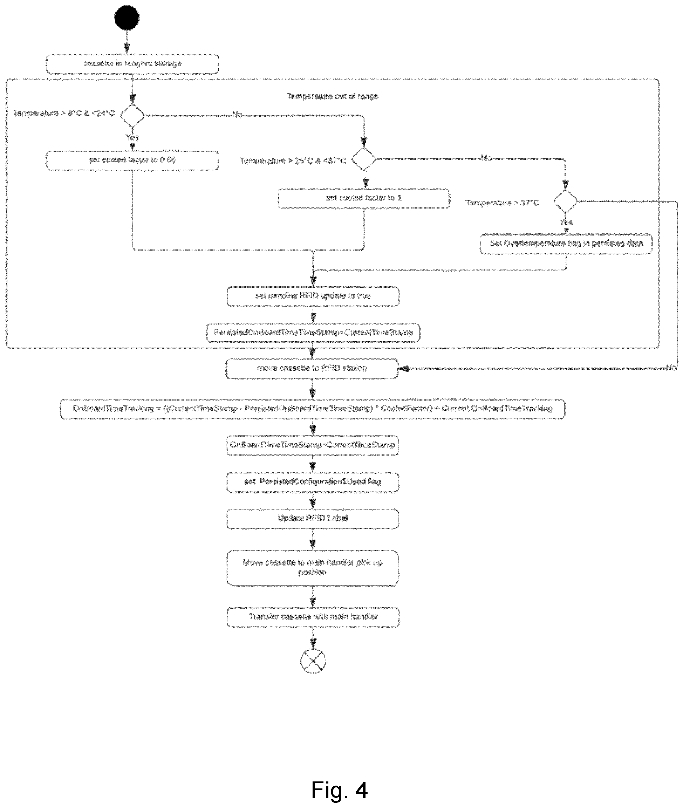

**Fig. 4** shows a flow chart of a method performed when the instrument transfers the cassette for processing. Again, the method begins with the cassette being in the reagent storage. A first determination is then made by the controller, as to whether the temperature of the reagent storage is >8°C and <24°C. If so, the cooled factor is set to 0.66. If not, the controller then determines if the temperature is >25°C and <37°C and, if so, the cooled factor is set to 1. If not, the controller then determines if the temperature is >37°C, and if so the overtemperature flag is set in the persisted data for that cassette. If not, the method moves to a step of moving the cassette to an RFID station.

However if the cooled factor is set (to 0.66 or 1) or the overtemperature flag is set, the method moves to a step in which a pending RFID update flag is set to true. Then, the PersistedOnBoardTimeStamp is set to the CurrentTimeStamp (the system timestamp). The method then moves to the step discussed above, where the cassette is moved to an RFID station. After it has, the method moves to a step in which the OnBoardTimeTracking is set equal to ((CurrentTimeStamp - PersistedOnBoardTimeStamp) * CooledFactor) + Current OnBoardTimeTracking.

The method then moves to a step in which the OnBoardTimeStamp is set equal to the current time stamp, and the PersistedConfiguration1 Used flag is set. Next, the RFID label of the cassette is updated, and the cassette is moved to the main handler (of the conveying system) and the main handler then transfers the cassette.

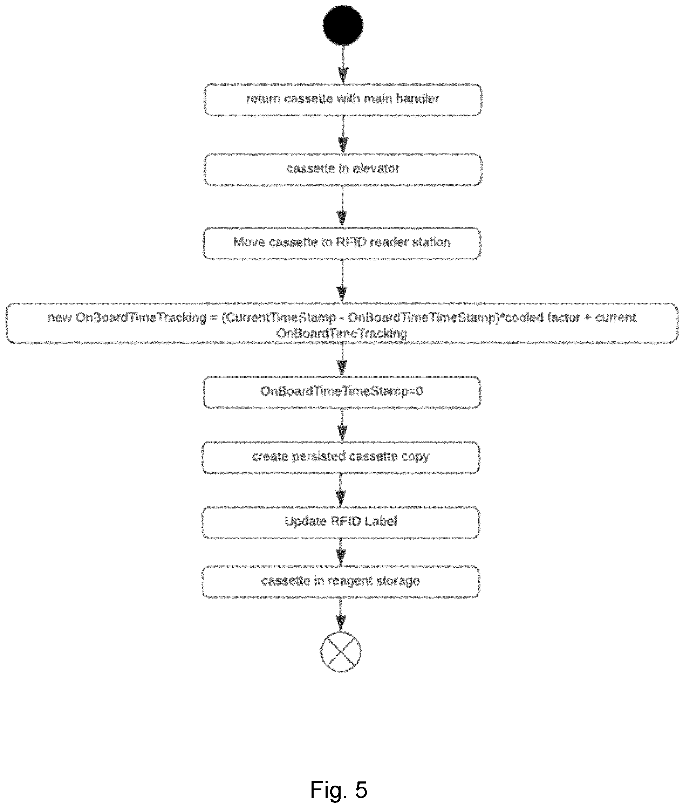

**Fig. 5** shows a flow chart of a method performed when the instrument returns a cassette from processing back to the reagent storage. It begins with the cassette being returned by the main handler, and so the method moves to a step in which the cassette is in an elevator of the conveying system. The cassette is then move to an RFID reader station, and the new onBoardTimeTracking value is set as (CurrentTimeStamp - onBoardTimeStamp)*cooled factor + current onBoardTimeTracking. The onBoardTimeStamp is set to zero in a next step, and then the persisted / cached cassette copy is created on the instrument. The method then moves to a step in which the RFID label is updated according to the new values, and then the cassette is stored in the reagent storage.

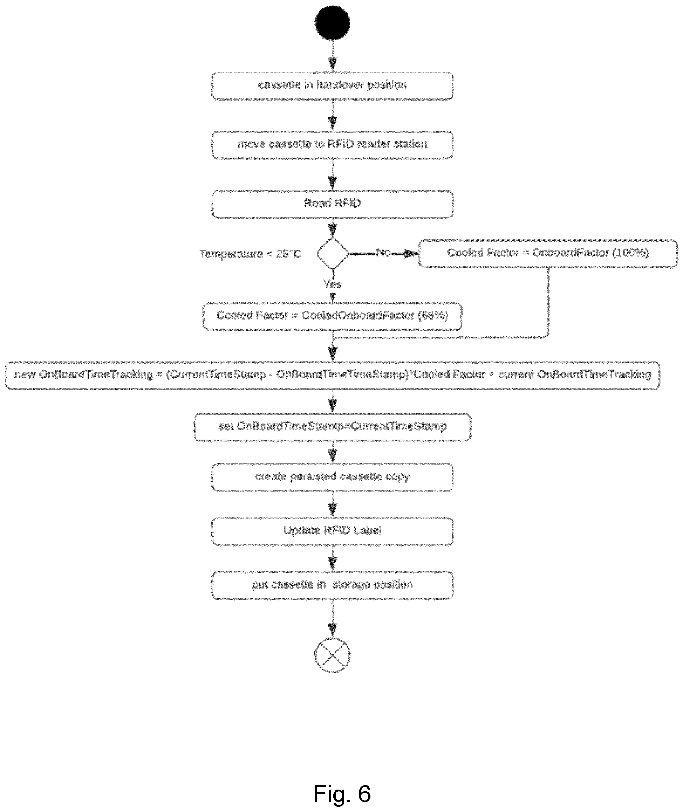

**Fig. 6** shows a flow chart of a method performed when the instrument stores cassettes in a processing module (e.g., the interim reagent storage) for sample preparation. It begins with the cassette in a handover position, after which the cassette is moved to an RFID reader station. The RFID tag on the cassette is then read, and the controller determines with the temperature of the reader station is <25°C. If it is, the cooled factor is set to 66% and if it is not the cooled factor is set to 100%. The method then moves to a step in which the controller calculates the new onBoardTimeTracking as equal to (CurrentTimeStamp - OnBoardTimeStamp)*cooled factor + current onBoardTimeTracking. The onBoardTimeStamp is then set to the CurrentTimeStamp, and the persisted / cached cassette copy is created by the instrument. Next, the RFID label of the cassette is updated using the new values and the cassette is placed in the storage position.

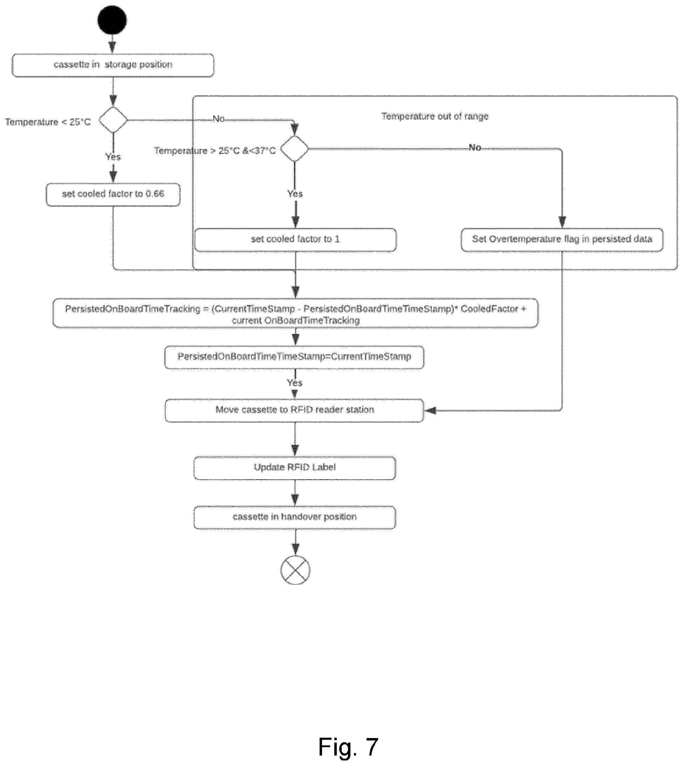

**Fig. 7** shows a flow chart of a method performed when the instrument unloads cassettes from the interim reagent storage. The method begins with the cassette in the storage position, and moves to a step where it is determined if the temperature is <25°C. if it is, Yes, the cooled factor is set to 0.66. If not, No, the method then moves on to determine if the temperature is >25°C and <37°C. If it is, Yes, the cooled factor is set to 1. If not, No, the method sets the Overtemperature flag in the persisted data and then moves directly to a step where the cassette is moved to an RFID reader station.

Going back, if the cooled factor is set to 0.66 or 1, the method moves to a step where the PersistedOnBoardTimeTracking is set to equal (CurrentTimeStamp - PersistedOnBoardTimeStamp)*CooledFactor + current OnBoardTimeTracking). The PersistedOnBoardTimeStamp is then set to equal the CurrentTimeStamp. The method then moves to the same step referred to above, where the cassette is moved to an RFID reader station. Thereafter, the RFID label of the cassette is updated, and the cassette is moved to the handover position.

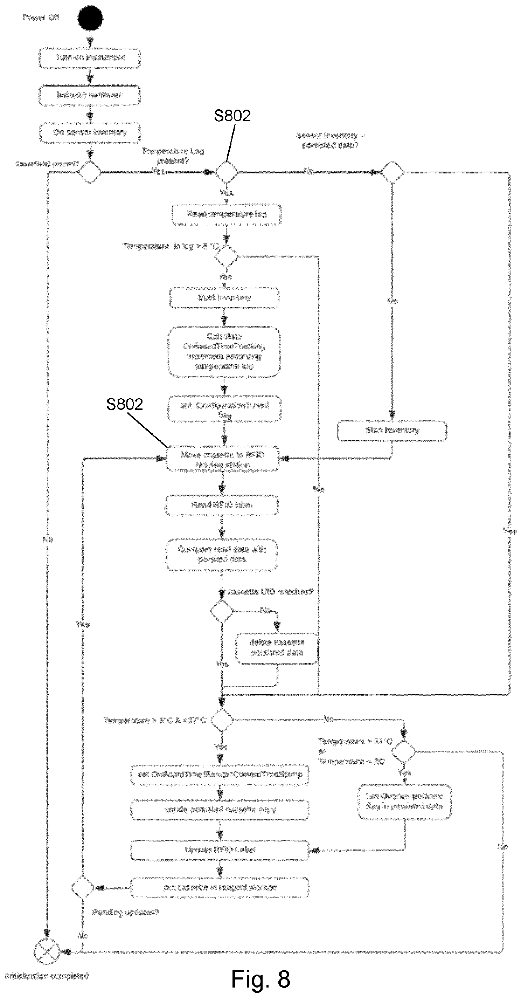

**Fig. 8** shows a flow chart of a method performed on start-up by the instrument. The method begins with the instrument being switched on, and the hardware then being initialised. Next, a sensor inventory is performed to determine if any cassettes are present in the reagent storage. If there are none, No, the method moves to the end and initialisation is completed. If there are, Yes, the method then moves to determine if a temperature log is present in step S802.

If the temperature log is present, Yes, it is read and it is then determined if the temperature recorded is >8°C. If it is, Yes, the inventory is started. The onBoardTimeTracking increment is calculated according to the temperatures in the temperature log. After, the Configuration1Used flag is set, and the method proceeds to step S804 whereupon the cassette is moved to the RFID reader station.

Going back, if the determination in step S802 was No (there is no temperature log) the method moves to a step where it is determined if the sensor inventory matches the persisted data stored in the machine (that is, for each cassette identified in the inventory, does persisted data exist for it). If not, No, the method then undertakes an inventor and then moves to step S802 where the cassette is moved to the RFID reading station.

After step S802 has been performed (according to any branch), the method then moves to a step in which the RFID label is read and the read data is compared with the persisted data. If the cassette unique ID (UID) in the persisted data does not match that from the read data, 'No', the method moves to a step where the cassette persisted data is deleted. If yes, and in any event after deleting the persisted data, the method moves to a temperature determination step where it is determined if the temperature is >8°C and less than 37°C. It should be noted that where the sensor inventory data does match the persisted data (as discussed earlier) the method then moves to this same temperature determination step.

If the temperature is within this range, the method moves to a step where the onBoardTimeStamp is set to equal the CurrentTimeStamp, the persisted / cached copy of the cassette tag/label data is created, and then the RFID label is updated. If the temperature is outside this range, No, next it is determined if the temperature is >37°C or <2°C, if not, No, the method moves to the initialisation complete step and ends. However, if it is, Yes, the Overtemperature flag is set in the persisted data, and then the method moves to the same update RFID label step as before.

Once the RFID label has been updated, the method moves to a step where the cassette is placed in the reagent storage. After this, it is determined if there are any pending updates. If there are, Yes, the method returns to Step 802 and proceeds as above. If not, No, the method moves to the initialisation complete step and ends.

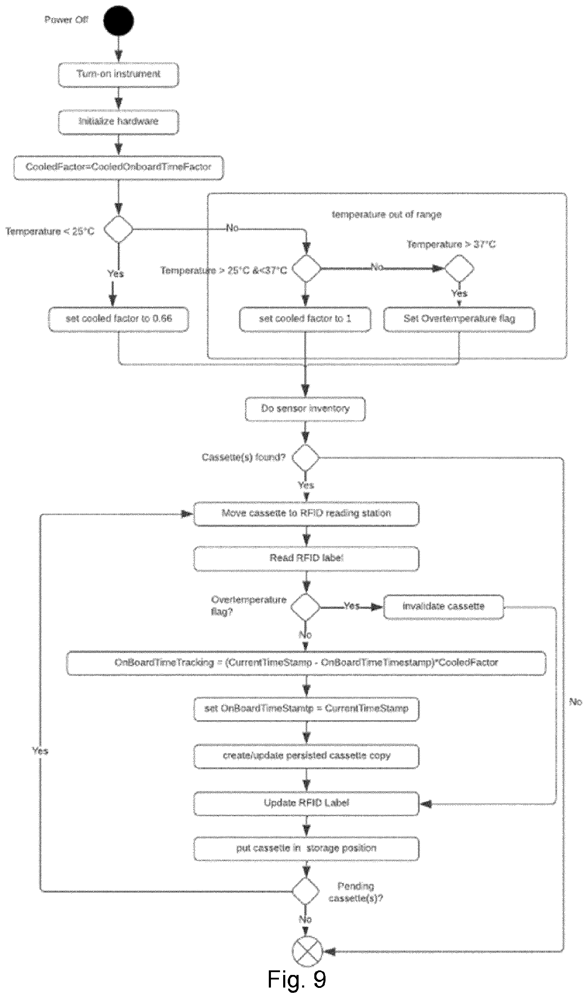

**Fig. 9** shows a flow chart of a method performed on start-up of the instrument. The method begins with the turn-on of the instrument, and the initialisation of the hardware. Then, the CooledFactor is set to the CooledOnBoardTimefactor. Next, it is determined if the temperature is <25°C, and if so the cooled factor is set to 0.66. If not, it is then determined if the temperature is >25°C and <37°C, and if so the cooled factor is set to 1. If not, it is then determined if the temperature is greater than 37°C, and if so the Overtemperature flag is set.

After this (when either the cooled factor or the overtemperature flag has been set), the method moves to a step in which a sensor inventory is performed it identify any cassettes within the interim reagent storage. If none are found, No, the method terminates. If any are found, Yes, the method moves to a step in which one of the identified cassettes is moved to the RFID reading station. The RFID label of that cassette is then read. It is then determined if the overtemperature flag has been set, and if so the cassette is invalidated (e.g., an invalid cassette flag is set). The method would then move to update the RFID label.

If the overtemperature flag has not been set, the method moves to a step where the OnBoardTimeTracking is set equal to the (CurrentTimeStamp - OnBoardTimeStamp)*CooledFactor). Once this has been set, the method moves to set the OnBoardTimeStamp to the CurrentTimeStamp, and the persisted cassette copy is either created or updated accordingly. The method then moves to the same update RFID label step as discussed above. Once the RFID label has been updated, the method moves to a step in which the cassette is put in the storage position. Then it is determined if there are any pending / remaining cassettes to be processed. If so, Yes, the method returns to the step of moving the next cassette to be processed to the RFID reading station and proceeds through the loop. When there are no pending / remaining cassettes, No, the method ends.

***

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

**Claims**

1. An automated laboratory instrument (100) for analysing biological samples, the automated laboratory instrument comprising:

   a plurality of reagent container holders (102a, 102b, 102c), each configured to hold a reagent container (104) containing reagent for use in the processing of a biological sample and including a tag (105) formed as a part thereof;
   a plurality of reagent container tag readers (106), each associated with a respective reagent container holder, and configured to read contents of the tag of a reagent container held within the respective reagent container holder; and
   a controller (101), configured to:

      cause one of the reagent container tag readers to read a timestamp from the tag of the reagent container;
      obtain a system timestamp; and
      update the timestamp stored in the tag utilising the obtained system timestamp.

2. The automated laboratory instrument of claim 1, wherein the controller is further configured to read an elapsed time value from the tag of the reagent container, and to update the elapsed time value obtained from the tag of the reagent container.

3. The automated laboratory instrument of claim 2, wherein the controller is configured to obtain a temperature of the reagent container or reagent container holder in which the reagent container is located, and wherein the update to the elapsed time value stored in the tag is based on the obtained temperature.

4. The automated laboratory instrument of claim 3, wherein the controller is configured to apply a temperature factor to a difference between the obtained system timestamp and the timestamp stored in the tag prior to updating the elapsed time value when the obtained temperature is within a first predefined range of temperatures.

5. The automated laboratory instrument of any one of claims 2-4, wherein the controller is configured to update the elapsed time value from the tag of the reagent container only when a temperature of the reagent container is greater than a lower threshold.

6. The automated laboratory instrument of any preceding claim, wherein the controller is configured, when a reagent container is placed in an instrument ingress reagent container holder of the plurality of reagent container holders, to set a usage flag stored in the tag of the reagent container.

7. The automated laboratory instrument of claim 6, wherein the usage flag which is set is selected from a plurality of usage flags, based on information held by the controller relating to the automated laboratory instrument.

8. The automated laboratory instrument of any preceding claim, wherein the controller is configured, when a reagent container is stored in a long-term reagent storage, to reset or remove the timestamp stored in the tag.

9. The automated laboratory instrument of any preceding claim, wherein the controller is further configured to determine whether the reagent container is invalid based on an elapsed time value read from the tag of the reagent container, and where it is, the controller is configured to set an invalid flag stored on the tag of the reagent container.

10. The automated laboratory instrument of any preceding claim, wherein the controller is configured to mirror at least some of the contents of the tag of the reagent container in memory and/or storage of the automated laboratory instrument.

11. The automated laboratory instrument of any preceding claim, wherein the controller is configured to monitor a temperature of one or more of the reagent container holders, and is configured to set an invalid tag stored on the tag of a reagent container stored in that reagent container holder when the temperature of the reagent container holder exceeds an acceptable upper temperature limit or is lower than an acceptable lower temperature limit.

12. The automated laboratory instrument of any preceding claim, wherein the controller is configured to monitor the removal of a reagent container from the automated laboratory instrument, and upon return of the same reagent container to the automated laboratory instrument, to update an elapsed time value stored on the tag of the reagent container to reflect the time during which the regent container was removed from the automated laboratory instrument.

13. The automated laboratory instrument of any preceding claim, wherein the controller is further configured to determine from an elapsed time value read from the tag of the reagent container, whether the reagent container can be used in a planned run.

14. The automated laboratory instrument of any preceding claim, wherein the controller is configured, during an initialisation phase, to detect the presence of one or more reagent containers within an interim reagent storage module, and for each of the detected reagent containers:

> obtain the system timestamp;
> read the timestamp and an elapsed time value from the tag of reagent container;
> update the elapsed time value stored on the tag; and
> update the timestamp stored on the tag.

15. A computer-implemented method, comprising the steps, on an automated laboratory instrument, of:

> reading, via a reagent container tag reader, a timestamp from a tag of a reagent container held in one of a plurality of reagent container holders;
> obtain a system timestamp; and
> update the timestamp stored in the tag utilising the obtained system timestamp.

Fig. 1

Fig. 2

Fig. 3

Fig. 4

Fig. 5

Fig. 6

Fig. 7

Fig. 8

Fig. 9

**EUROPEAN SEARCH REPORT**

Application Number

EP 24 21 8774

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/266679 A1 (FRANK PAUL [CH] ET AL) 24 September 2015 (2015-09-24) * paragraphs [0002], [0030] - [0040], [0062] - [0068], [0121], [0129] * ----- | 1-15 | INV.<br>G16H10/40<br>B01L3/00<br>G01N35/00<br>G16H40/63 |
| X | US 2013/280129 A1 (WATANABE ATSUSHI [JP] ET AL) 24 October 2013 (2013-10-24) * paragraphs [0019], [0029], [0036], [0037], [0038], [0052] - [0069]; figure 3 * ----- | 1-15 | |
| X | US 2008/024301 A1 (FRITCHIE PATRICK P [US] ET AL) 31 January 2008 (2008-01-31) | 1,2,15 | |
| A | * paragraphs [0015] - [0017], [0043], [0050] * ----- | 3-14 | |
| X | US 2024/003925 A1 (KOCHAR MANISH [US] ET AL) 4 January 2024 (2024-01-04) | 1,2,15 | |
| A | * summary; paragraph [0426] * ----- | 3-14 | |

TECHNICAL FIELDS
SEARCHED (IPC)

G16H
G01N
B01L

The present search report has been drawn up for all claims

| Place of search | Date of completion of the search | Examiner |
|---|---|---|
| Munich | 16 May 2025 | Huber, Alexander |

EPO FORM 1503 03.82 (P04C01)

## ANNEX TO THE EUROPEAN SEARCH REPORT
## ON EUROPEAN PATENT APPLICATION NO.

EP 24 21 8774

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2025

| Patent document cited in search report | | Publication date | Patent family member(s) | | Publication date |
|---|---|---|---|---|---|
| US 2015266679 | A1 | 24-09-2015 | EP | 2455765 A1 | 23-05-2012 |
| | | | US | 2012294766 A1 | 22-11-2012 |
| | | | US | 2015266679 A1 | 24-09-2015 |
| | | | US | 2015268260 A1 | 24-09-2015 |
| US 2013280129 | A1 | 24-10-2013 | CN | 103384834 A | 06-11-2013 |
| | | | EP | 2647997 A1 | 09-10-2013 |
| | | | JP | 5643339 B2 | 17-12-2014 |
| | | | JP | WO2012073878 A1 | 19-05-2014 |
| | | | US | 2013280129 A1 | 24-10-2013 |
| | | | WO | 2012073878 A1 | 07-06-2012 |
| US 2008024301 | A1 | 31-01-2008 | CA | 2659597 A1 | 31-01-2008 |
| | | | EP | 2069801 A2 | 17-06-2009 |
| | | | EP | 3196650 A1 | 26-07-2017 |
| | | | ES | 2620817 T3 | 29-06-2017 |
| | | | JP | 6148698 B2 | 14-06-2017 |
| | | | JP | 2009544972 A | 17-12-2009 |
| | | | JP | 2013238607 A | 28-11-2013 |
| | | | JP | 2015200668 A | 12-11-2015 |
| | | | US | 2008024301 A1 | 31-01-2008 |
| | | | WO | 2008014117 A2 | 31-01-2008 |
| US 2024003925 | A1 | 04-01-2024 | AU | 2016297652 A1 | 15-02-2018 |
| | | | AU | 2022231693 A1 | 06-10-2022 |
| | | | AU | 2023204139 A1 | 20-07-2023 |
| | | | AU | 2024264661 A1 | 05-12-2024 |
| | | | CA | 2992983 A1 | 26-01-2017 |
| | | | CA | 3171618 A1 | 26-01-2017 |
| | | | CN | 108139390 A | 08-06-2018 |
| | | | CN | 110139953 A | 16-08-2019 |
| | | | CN | 113358860 A | 07-09-2021 |
| | | | CN | 117012359 A | 07-11-2023 |
| | | | EP | 3329277 A1 | 06-06-2018 |
| | | | EP | 4465206 A2 | 20-11-2024 |
| | | | JP | 6903735 B2 | 14-07-2021 |
| | | | JP | 6981975 B2 | 17-12-2021 |
| | | | JP | 7160998 B2 | 25-10-2022 |
| | | | JP | 7478127 B2 | 02-05-2024 |
| | | | JP | 7541062 B2 | 27-08-2024 |
| | | | JP | 2018524616 A | 30-08-2018 |
| | | | JP | 2019525171 A | 05-09-2019 |
| | | | JP | 2021156902 A | 07-10-2021 |
| | | | JP | 2022024111 A | 08-02-2022 |
| | | | JP | 2023012476 A | 25-01-2023 |
| | | | JP | 2024112803 A | 21-08-2024 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 24 21 8774

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2025

| Patent document cited in search report | Publication date | Patent family member(s) | | Publication date |
|---|---|---|---|---|
| | | JP | 2024178158 A | 24-12-2024 |
| | | KR | 20180044289 A | 02-05-2018 |
| | | KR | 20190083322 A | 11-07-2019 |
| | | KR | 20220092648 A | 01-07-2022 |
| | | KR | 20240052891 A | 23-04-2024 |
| | | KR | 20240156646 A | 30-10-2024 |
| | | US | 2019391170 A1 | 26-12-2019 |
| | | US | 2020105409 A1 | 02-04-2020 |
| | | US | 2021389338 A1 | 16-12-2021 |
| | | US | 2022365106 A1 | 17-11-2022 |
| | | US | 2024003925 A1 | 04-01-2024 |
| | | US | 2024094233 A1 | 21-03-2024 |
| | | WO | 2017015636 A1 | 26-01-2017 |

EPO FORM P0459

For more details about this annex : see Official Journal of the European Patent Office, No. 12/82

page 2 of 2